Europäisches Patentamt

European Patent Office

Office européen des brevets

(11) Publication number: **0 481 215 A1**

# EUROPEAN PATENT APPLICATION

(21) Application number: **91115384.9**

(22) Date of filing: **11.09.91**

(51) Int. Cl.5: **C12Q 1/68**, C12Q 1/70

(30) Priority: **17.10.90 JP 279589/90**

(43) Date of publication of application:
**22.04.92 Bulletin 92/17**

(84) Designated Contracting States:
**AT BE CH DE DK ES FR GB GR IT LI LU NL SE**

(71) Applicant: **SHIONOGI SEIYAKU KABUSHIKI KAISHA trading under the name of SHIONOGI & CO. LTD.**
**1-8, Doshomachi 3-chome Chuo-ku Osaka 541(JP)**

(72) Inventor: **Igarashi, Hisanaga**
**3-1-1-801, Furuichi, Joto-ku**
**Osaka-shi, Osaka(JP)**
Inventor: **Aono, Yuko**
**5F-808, Misawa-cho**
**Ibaraki-shi, Osaka(JP)**
Inventor: **Sato, Akihiko**
**2-3-21, Minamiminohara**
**Ibaraki-shi, Osaka(JP)**

(74) Representative: **Vossius & Partner**
**Siebertstrasse 4 P.O. Box 86 07 67**
**W-8000 München 86(DE)**

(54) **Method for the detection of an HIV-1 genome by a two-step PCR method and oligonucleotide used in the same.**

(57) The present invention relates to a method for the high-sensitive and high-specific detection of an HIV-1 genome by a two-step PCR method and to an oligonucleotide specific for HIV-1, which is used in this method.

EP 0 481 215 A1

EP 0 481 215 A1

The present invention relates to a method for the high-sensitive and high-specific detection of an HIV-1 genome by a two-step PCR method, and it also relates to an oligonucleotide specific for HIV-1, which is used in this method.

It has been known that human immunodeficiency viruses (HIVs) are classified into two types, i.e. HIV-1 and HIV-2. In particular, HIV-1 is extremely raging throughout the world as a pathogen of acquired immunodeficiency syndrome (AIDS).

At present, serological detection of antibodies against HIV has been most frequently utilized for the diagnosis of HIV infection. However, there are serious problems that a decision as being antibody positive does not always correspond to the presence of HIV since an antibody titer has not yet increased the initial stage of infection in spite of the presence of infectious viruses. For this reason, other methods such as an HIV antigen detection and a virus isolation have been also utilized, although these methods do not have a satisfactory accuracy, which is a problem to be solved.

Recently, it has become possible to detect the presence of HIV with high sensitivity by amplifying HIV provirus DNA in peripheral blood leukocytes according to a polymerase chain reaction (PCR) method, and therefore, the above-mentioned problems are going to be solved (Kwok, S. et al., J. Virology 61(5), 1690-1694, 1987, Ou, C-Y. et al., Science 239, 295-297, 1988; Byrne, B. et al., Nucleic Acids Research 16(9), 4165, 1988; Hart, C. et al., Lancet 596-599, 1988; Rayfield, M. et al., J. Inf. Dis. 158(6), 1170-1175, 1988; Simmonds, P. et al., J. Virology 64(2), 846-872, 1990, Oka, S. et al., BBRC 167(1), 1990; and Pang, S. et al., Nature 343, 85-89, 1990).

However, there is a problem which of the HIV genes the primers used in PCR are selected from, because a high degree of variability is observed in the HIV genes as an important property of HIV. As can be seen from the previous reports, three regions, i.e., LTR, gag, and env, have been used to primer PCR reactions. Because it was found that env is the most variable region, this region seems to be unsuitable for the purpose of examining clinical specimens or the like. On the other hand, LTR and gag have been usually used, because they are highly conserved regions.

As previously described, it has been known that HIVs are classified into two types, i.e. HIV-1 and HIV-2. In particular, HIV-1 is extremely raging throughout the world as a pathogen of AIDS. Therefore, it is especially important to detect HIV-1 specifically and distinctively from another type.

Thus, the technical problem underlying the present invention was to establish a system for the specific PCR detection of HIV-1, thus distinguishing between HIV-1 and HIV-2. The solution to this technical problem is provided by the embodiments characterized in the claims. Accordingly, the present invention provides a method for the detection of HIV-1 with high specificity and high sensitivity, and it also provides oligonucleotides specific for HIV-1, which are used in this method.

The present invention is concerned with the vpr-tat-rev which is an essentially different gene region between HIV-1 and HIV-2 on the basis of the genome analysis thereof. It was found that a specific detection of HIV-1 can be achieved by selecting oligonucleotides from this region and using the oligonucleotides as primers to conduct the two-step PCR method (Japanese Patent Application No.2-206290).

Accordingly, the present invention relates to:

(1) A method for the detection of an HIV-1 genome by a two-step PCR method;

(2) A method for the detection of an HIV-1 genome as recited in Item (1), wherein a primer set is selected from a vpr-tat-rev region and is capable of specifically detecting an HIV-1 genome;

(3) A method for the detection of an HIV-1 genome as recited in Item (2), wherein said primer set comprises oligonucleotides (a) to (d), each of which has a nucleotide sequence of 15 to 40 bases containing at least 15 consecutive bases in the nucleotide sequence shown by one of the following sequences (a) to (d):

(a) 5'GGGTGTCGACATAGCAGAATAGGCGTTACT3' (SEQ ID NO. 1)

(b) 5'ATGGAGCCAGTAGATCCTAGACTAGAGCCC3' (SEQ ID NO. 2)

(c) 5'CGCTTCTTCCTGCCATAGGAGATGCCTAAG3' (SEQ ID NO. 3)

(d) 5'AGGAGGTCTTCGTCGCTGTCTCCGCTTCTT3' (SEQ ID NO. 4)

wherein said oligonucleotides (a) and (d) are used in the first-step PCR and said oligonucleotides (b) and (c) are used in the second-step PCR;

(4) A method for the detection of an HIV-1 genome as recited in any one of Items (1) to (3), wherein the

2

primer concentration in the first-step PCR of said two-step PCR method is in the range of from 5 to 25 nM; and,

(5) An oligonucleotide selected from the group consisting of oligonucleotides (a) to (d), each of which has a nucleotide sequence of 15 to 40 bases containing at least 15 consecutive bases in the nucleotide sequence shown by one of the following sequences (a) to (d):

(a) 5'GGGTGTCGACATAGCAGAATAGGCGTTACT3' (SEQ ID NO. 1)

(b) 5'ATGGAGCCAGTAGATCCTAGACTAGAGCCC3' (SEQ ID NO. 2)

(c) 5'CGCTTCTTCCTGCCATAGGAGATGCCTAAG3' (SEQ ID NO. 3)

(d) 5'AGGAGGTCTTCGTCGCTGTCTCCGCTTCTT3' (SEQ ID NO. 4)

As used herein, oligonucleotide (a) refers to an oligonucleotide having a nucleotide sequence of at least 15 continuous bases in the nucleotide sequence shown by the above-identified sequence (a), and having a strand length of 15 to 40 bases; oligonucleotide (b) refers to an oligonucleotide having a nucleotide sequence of at least 15 continuous bases in the nucleotide sequence shown by the above-identified sequence (b), and having a strand length of 15 to 40 bases; oligonucleotide (c) refers to an oligonucleotide having a nucleotide sequence of at least 15 continuous bases in the nucleotide sequence shown by the above-identified sequence (c), and having a strand length of 15 to 40 bases; and oligonucleotide (d) refers to an oligonucleotide having a nucleotide sequence of at least 15 continuous bases in the nucleotide sequence shown by the above-identified sequence (d), and having a strand length of 15 to 40 bases. These oligonucleotides can be sufficiently used as a primer set capable of specifically detecting an HIV-1 genome, even when they have a strand length of as short as 15 bases and when they have a strand length of as long as 40 bases.

The figures show:

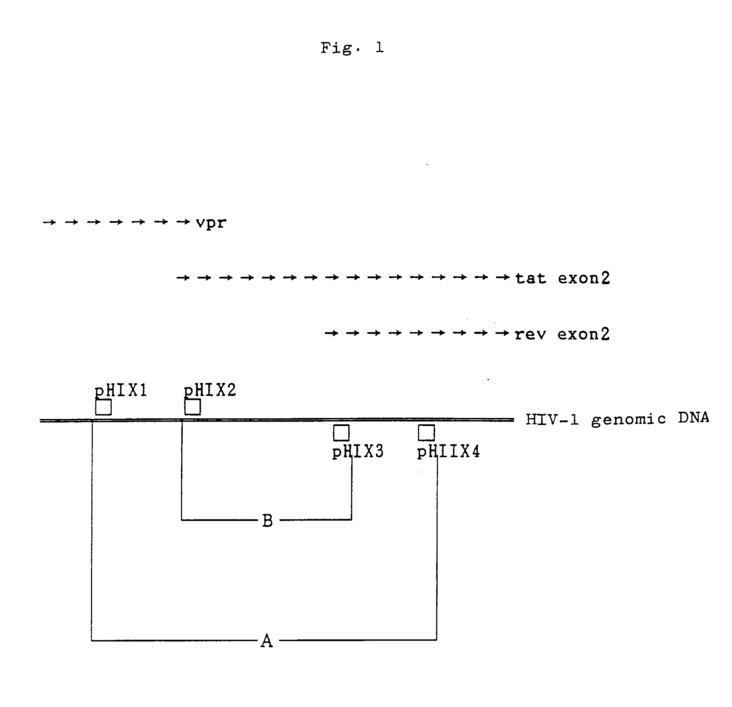

Figure 1 : a relative position of vpr, tat exon 2, and rev exon 2 regions, as well as relative positions of primers used in the two-step PCR method.

In the present invention, as a method for the specific amplification of an HIV-1 genomic DNA, a two-step PCR method was used. The polymerase chain reaction (PCR) method refers to a method for the amplification of a particular DNA fragment based upon the fact that primers are required for DNA polymerase. This is based on the principle that with the use of two primers (oligonucleotides) designed for a target portion of the base sequence, in the presence of four deoxyribonuceoside triphosphates [dNTPs (dATP, dCTP, dGTP, and dTTP)], the second strand in the portion interposed between the two primers is synthesized by DNA polymerase with the first strand of the double-strand DNA as a template. In the actual method, particular DNAs are amplified by repeating the following processes as one cycle up to the number of cycles required, under the optimum conditions of temperature and duration; the conversion of doublestranded DNAs into a single-stranded form by thermal denaturation (denaturation process); annealing of primers to single-stranded template DNAs (annealing process); and synthesis of complementary DNAs by DNA polymerase (extension process). The two-step PCR method, in which the above-mentioned PCR method is conducted twice, is characterized in that the primer concentration in the first step is set lower than the ordinary concentration of 1 $\mu$M on the scale of 1 : 40 to 200. Thus, it is possible to amplify a target DNA more sensitively and more specifically as compared with conventional PCR methods.

In the present invention, five kinds of HIV-1 infected cell strain DNAs are used as template DNAs, and HIV-2 clonal plasmid DNA, HTLV-1 infected cell line DNA, and HTLV-2 clonal plasmid DNA are used as control specimens.

As the primer set, the following oligonucleotides are synthesized by an automatic synthesizer and used: 15- to 40-mer oligonucleotide having a nucleotide sequence of at least 15 continuous bases in the nucleotide sequence shown by above-identified sequence (a), and preferably 30-mer oligonucleotide (pHIX1) shown by above-identified sequence (a); 15-to 40-mer oligonucleotide having a nucleotide sequence of at least 15 continuous bases in the nucleotide sequence shown by above-identified sequence (b), and preferably 30-mer oligonucleotide (pHIX2) shown by above-identified sequence (b); 15- to 40-mer oligonucleotide having a nucleotide sequence shown by above-identified sequence (c), and preferably 30-mer oligonucleotide (pHIX3) shown by above-identified sequence (c); and 15- to 40-mer oligonucleotide having a nucleotide sequence of at least 15 continuous bases in the nucleotide sequence shown by above-identified sequence (d), and preferably 30-mer oligonucleotide (pHIX4) shown by above-identified sequence (d). This primer set is used in such a manner that primers pHIX1 and pHIX4 are used in the first-step PCR and primers pHIX2 and pHIX3 are used in the second-step PCR. The primer concentration used in the first-step PCR is in the range of from 10 to 25 nM, and that used in the second step is an ordinary one, for example, in the range of from 0.5 to 1 $\mu$M.

The conditions of PCR are as described below. In the first-step PCR, the denaturation process is conducted at 92 to 96°C for 0.5 to 2 min, and preferably at 94°C for 1 min; the annealing process is conducted at 50 to 72°C for 0.5 to 2 min, preferably at 60°C for 1 min; the extension process is conducted at 70 to 74°C for 1 to 3 min, and preferably at 72°C for 2 min; and these processes as one cycle are repeated in 20 to 30 cycles and preferably in 25 cycles.

In the second-step PCR, the denaturation process is conducted at 92 to 96°C for 0.5 to 2 min, and preferably at 94°C for 1 min; the annealing process is conducted at 50 to 72°C for 0.5 to 2 min, and preferably at 65°C for 1 min; the extension process is conducted at 70 to 74°C for 1 to 3 min, and preferably at 72°C for 2 min; and these processes as one cycle are repeated in 25 to 35 cycles and preferably in 30 cycles.

The thermostable DNA polymerase is used in 2 to 3 units and preferably in 2.5 units per assay for each of the first and second steps.

The amplified DNA fragments are detected by electrophoresis on a mixed gel of agarose and Nusieve (trade name, Takara Shuzo) together with molecular size markers, followed by ethidium bromide staining and then ultraviolet irradiation.

The examples illustrate the invention.

Example-1 (Preparation of template DNAs)

Each of the various DNAs described below was used as a template DNA. The template DNA in Item (e) was used as a diluent to make the DNA concentration constant and as a negative control specimen.

(a) HIV-1 infected cells:

① Molt-4 cells infected with HTLV-IIIb (Matsuyama, T. et al., J. Virology 63, 2504-2509, 1989) (isolated from patients in the United States);

② Molt-4 cells infected with LAV-1 (F. Barre-Sinoussi et al., Science 220, 868-870, 1983) (isolated from patients in the United States);

③ Molt-4 cells infected with HIV-1 #8 (isolated from patients in Kenya);

④ Molt-4 cells infected with HIV-1 #15 (isolated from patients in Kenya);

⑤ Molt-4 cells infected with HIV-1 #17 (isolated from patients in Kenya); and

⑥ Molt-4 cells infected with HIV-1 (IMS-2) (isolated from patients in Japan);

High-molecular DNA of these cells: 100 ng/$\mu$l;

(b) HIV-2 clonal plasmid DNA (GH1) (Hasegawa, A. et al., AIDS research and Human retroviruses 5, 593-604, 1989);

(c) High-molecular DNA of the HTLV-1 infected cell strain (TL-Su, (Sugamura, K. et al., Int. J. Cancer 34, 221-228, 1984)]: 100 ng/$\mu$l:

(d) HTLV-2 clonal plasmid DNA (pH6-B3.5) (Shimotohno, K. et al., Proc. Natl. Acad. Sci. USA 82, 3101-3105, 1985); and

(e) High-molecular DNA of the control cell line which is negative for all of HTLV-1, HTLV-2, HIV-1, and HIV-2 [human glioblastoma cell line; A172, (Igarashi, H. et al., Oncogene 1, 79-85, 1987)]: 100 ng/$\mu$l

Example-2 (Preparation of primers)

As primers used in the two-step PCR for HIV-1, the following oligonucleotides were synthesized by an automatic synthesizer (Cyclone, Biosearch Co.). The base sequence, position (nt), and length of the respective primers are described below (Ratner, L. et al., Nature 313, 277-284, 1985).

```
pHIX1 : 5'GGGTGTCGACATAGCAGAATAGGCGTTACT3' (SEQ ID NO. 1)
           nt; 5781-5810,   30mer

pHIX2 : 5'ATGGAGCCAGTAGATCCTAGACTAGAGCCC3' (SEQ ID NO. 2)
           nt; 5830-5859,   30mer

pHIX3 : 5'CGCTTCTTCCTGCCATAGGAGATGCCTAAG3' (SEQ ID NO. 3)
           nt; 5955-5984,   30mer

pHIX4 : 5'AGGAGGTCTTCGTCGCTGTCTCCGCTTCTT3' (SEQ ID NO. 4)
           nt; 5977-6006,   30mer
```

These primers are positioned, as shown in Figure 1, to prime within the vpr, tat exon 2 and rev exon 2 regions. This was determined by comparing the DNA sequences of five HIV-1 clones [pNL432 (Adachi, A. et al., J. Virology 59(2), 284-291, 1986), HTLV-3/LAV (Adachi, A. et al.; supra), ARV-2 (Sanchez-Pescador, R. et al., Science 227, 484-492, 1985), BH10 (Ratner, L. et al.; supra), BRU (Wain-Hobson, S. et al., Cell 40, 9-17, 1985)] and selecting out a more common sequence.

Example-3 (Two-step PCR method)

For PCR, the GeneAmp Kit (PERKIN ELMER CETUS; Takara Shuzo) was used, and the reaction was conducted using the PERKIN ELMER CETUS DNA Thermal Cycler.

The template DNAs in Item (a) of Example-1 were used as a specimen in ten fold serial dilutions($10^{-1}$ to $10^{-7}$) with the template DNA in Item (e). The template DNAs in Items (c) and (e) were used at an amount of 0.5 μg, and those in Items (b) and (d) were used at an amount of 10 ng. These template DNAs in Items (b), (c), (d), and (e) were used as a control specimen for examining a specificity.

The respective primer sets prepared in Example-2 are used as shown in Figure 1; a combination of pHIX1 and pHIX4 is used for amplifying the DNA of size A, while a combination of pHIX2 and pHIX3 is used for amplifying the DNA of size B. From the primers pHIX1 to pHIX4, pHIX1 and pHIX2 are sense primers, while pHIX3 and pHIX4 are anti-sense primers.

(1) First-step PCR
- Each of the template DNAs in a serial dilution: 5 μl
  (corresponding to 0.5 μg of DNA),
- 10 × reaction buffer: 10 μl
  [500 mM KCl, 100 mM Tris-HCl (pH 8.3), 15 mM MgCl$_2$, 0.1% (w/v) gelatin],
- dNTPs Mix: 16 μl
  (1.25 mM dNTPs (dATP, dCTP, dGTP, and dTTP)],
- Primers (pHIX1 and pHIX4): 10 nM,
- Taq DNA polymerase: 2.5 units/assay.

These were mixed together, and the total volume of the solution was adjusted to 100 μl.

The PCR conditions are as follows:

Denaturation process: 94°C, 1 min

Annealing process : 60°C, 1 min

Extension process : 72°C, 2 min

These processes as one cycle were repeated in 25 cycles.

(2) Second-step PCR
- Template DNAs: 10 μl;
  (1/10 volume of the PCR products obtained in the first-step PCR),
- 10 × reaction buffer: 10 μl,
- dNTPs Mix (1.25 mM dNTPs): 16 μl,
- Primers (pHIX2 and pHIX3): 1.0 μM;
- Taq DNA polymerase: 2.5 units/assay.

These were mixed together, and the total volume of the solution was adjusted to 100 μl.

The PCR conditions are as follows:

Denaturation process: 94°C, 1 min

Annealing process : 65°C, 1 min

Extension process : 72°C, 2 min

These processes as one cycle were repeated in 30 cycles.

(3) Detection by gel electrophoresis and ethidium bromide staining

First, 1/10 volume, 10 $\mu$l, of the product obtained in the second-step PCR was subjected to electrophoresis on a mixed gel of 2.0% Nusieve (trade name, Takara Shuzo) and 1.0% agarose together with DNA molecular weight markers, followed by ethidium bromide staining. The stained image was obtained by ultraviolet irradiation, and photographed with a Polaroid film, followed by the judgement of detection. The theoretical target size of the second-step PCR products is 155 bps.

Results

(1) The two-step PCR was conducted using the DNA of HIV-1 infected cells ① in Item (a) of Example-1, as a template DNA. As a result, a specific-size band (155 bps) was distinctly observed up to a dilution of $10^{-5}$.

(2) The two-step PCR was conducted using each of the DNAs of HIV-2, HTLV-1, and HTLV-2 in Item (b), (c), and (d) of Example-1, respectively, as a template DNA. As a result, all of the cases were negative and no bands were observed.

(3) The two-step PCR was conducted using each of the DNAs of five HIV-1 clones ②, ③, ④, ⑤, and ⑥ in Item (a) of Example-1, as a template DNA. As a result, a specific-size band (155 bps) was distinctly observed up to a dilution of $10^{-6}$ in case ⑤, and up to a dilution of $10^{-5}$ in the other cases, similarly to the result obtained in Item (1).

The origin of each of the template DNAs is as follows. The DNAs of clones ① and ② are isolated from patients in United States; the DNAs of clones ③, ④, and ⑤ are isolated from patients in Africa; and the DNA of clone ⑥ is isolated from patients in Japan.

Discussion

From result (1), it was possible to conduct the specific detection up to a dilution of $10^{-5}$. Although the precise number of HIV-1 genomes contained in each of the HIV-1 infected cells used herein is unknown, it is found by calculation that there are 0.5 HIV-1 infected cells in 5 $\mu$l (0.5 $\mu$g) of a $10^{-5}$ dilution on the basis of the fact that the amount of DNAs contained in $10^5$ human cells corresponds to about 1 $\mu$g. Accordingly, it is possible to detect 0.5 or more infected cells, and a sufficient sensitivity of detection can be obtained, in this case.

From result (2), it was found that the DNAs of HIV-2, HTLV-1, and HTLV-2 cannot be amplified by PCR using oligonucleotides of this invention as a primer. Since a sufficient amount of DNAs were used as a template in all the experiments, it is concluded that this result is due to the high specificity of the primers, but not due to the low sensitivity of the method. That is to say, the primers defined herein by the present inventors are specific only for HIV-1 DNA.

Further, from results (1) and (3), it was confirmed that HIV-1 clones isolated from various areas such as Africa, United States, and Japan, can be detected with a similar sensitivity and a similar specificity. Therefore, it is considered that the primers defined herein can be satisfactory used as a universal primer for the detection of HIV-1 by PCR.

## SEQUENCE LISTING

INFORMATION FOR SEQ ID NO:1:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: no

    (xi) SEQUENCE DESCRIPTION: SEQ ID NO:1:

GGGTGTCGAC ATAGCAGAAT AGGCGTTACT         30

INFORMATION FOR SEQ ID NO:2:

    (i) SEQUENCE CHARACTERISTICS:

        (A)LENGTH: 30 base pairs

        (B)TYPE: nucleic acid

        (C)STRANDEDNESS: single

        (D)TOPOLOGY: linear

    (ii) MOLECULE TYPE: other nucleic acid, synthesized DNA

    (iv) ANTI-SENSE: no

    (xi) SEQUENCE DESCRIPTION: SEQ ID NO:2:

ATGGAGCCAG TAGATCCTAG ACTAGAGCCC         30

INFORMATION FOR SEQ ID NO:3:

    (i) SEQUENCE CHARACTERISTICS:

        (A)LENGTH: 30 base pairs

(B)TYPE: nucleic acid

(C)STRANDEDNESS: single

(D)TOPOLOGY: linear

(ii) MOLECULE TYPE: other nucleic acid, synthesized DNA

(iv) ANTI-SENSE: yes

(xi) SEQUENCE DESCRIPTION: SEQ ID NO:3:

CGCTTCTTCC TGCCATAGGA GATGCCTAAG                                    30

INFORMATION FOR SEQ ID NO:4:

        (i) SEQUENCE CHARACTERISTICS:

            (A)LENGTH: 30 base pairs

            (B)TYPE: nucleic acid

            (C)STRANDEDNESS: single

            (D)TOPOLOGY: linear

        (ii) MOLECULE TYPE: other nucleic acid, synthesized DNA

        (iv) ANTI-SENSE: yes

        (xi) SEQUENCE DESCRIPTION: SEQ ID NO:4:

AGGAGGTCTT CGTCGCTGTC TCCGCTTCTT                                    30

## Claims

1. A method for the detection of an HIV-1 genome by a two-step PCR method.

2. The method of claim 1, wherein a primer set is selected from a vpr-tat-rev region and is capable of specifically detecting an HIV-1 genome.

3. The method of claim 2, wherein said primer set comprises oligonucleotides (a) to (d), each of which has a nucleotide sequence of 15 to 40 bases containing at least 15 consecutive bases in the nucleotide sequence shown by one of the following sequences (a) to (d):

8

(a) 5'GGGTGTCGACATAGCAGAATAGGCGTTACT3'

(b) 5'ATGGAGCCAGTAGATCCTAGACTAGAGCCC3'

(c) 5'CGCTTCTTCCTGCCATAGGAGATGCCTAAG3'

(d) 5'AGGAGGTCTTCGTCGCTGTCTCCGCTTCTT3'

wherein said oligonucleotides (a) and (d) are used in the first-step PCR and said oligonucleotides (b) and (c) are used in the second-step PCR.

4. The method of any one of claims 1 to 3, wherein the primer concentration in the first-step PCR of said two-step PCR method is in the range of from 5 to 25 nM.

5. An oligonucleotide selected from the group consisting of oligonucleotides (a) to (d), each of which has a nucleotide sequence of 15 to 40 bases containing at least 15 consecutive bases in the nucleotide sequence shown by one of the following sequences (a) to (d):

(a) 5'GGGTGTCGACATAGCAGAATAGGCGTTACT3'

(b) 5'ATGGAGCCAGTAGATCCTAGACTAGAGCCC3'

(c) 5'CGCTTCTTCCTGCCATAGGAGATGCCTAAG3'

(d) 5'AGGAGGTCTTCGTCGCTGTCTCCGCTTCTT3'

Fig. 1

→ → → → → → → vpr

→ → → → → → → → → → → → → → → tat exon2

→ → → → → → → → → rev exon2

pHIX1    pHIX2

HIV-1 genomic DNA

pHIX3    pHIIX4

B

A

European
Patent Office

EUROPEAN SEARCH
REPORT

Application Number

EP 91 11 5384

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PROCEEDINGS OF THE NATIONAL ACADEMY OF SCIENCES vol. 86, April 1989, WASHINGTON D.C. USA pages 2423 - 2427; D.J.KEMP ET AL.: 'COLORIMERTIC DETECTION OF SPECIFIC DNA SEGMENTS AMPLIFIED BY POLYMERASE CHAIN REACTIONS' * the whole document * * | 1,4 | C 12 Q 1/68 C 12 Q 1/70 |
| A | EP-A-0 201 184 (CETUS CORPORATION) * page 19 - page 20; example 10 * * | 1 | |
| P,A | EP-A-0 403 333 (INSTITUT PASTEUR) * page 4, line 45 - page 5, line 5 * * | 1-5 | |
| A | CELL vol. 58, September 1989, CAMBRIDGE US. pages 901 - 910; A. MEYERHANS ET AL: 'TEMPORAL FLUCTUATIONS IN HIV QUASISPECIES IN VIVO ARE NOT REFLECTED BY SEQUENTIAL HIV ISOLATIONS' * page 908, left column, line 47 - line 56 * * | 3,5 | |

TECHNICAL FIELDS
SEARCHED (Int. Cl.5)

C 12 Q

The present search report has been drawn up for all claims

| Place of search | Date of completion of search | Examiner |
|---|---|---|
| The Hague | 10 February 92 | OSBORNE H.H. |